# EUROPEAN PATENT SPECIFICATION

(11) **EP 1 292 660 B1**
(45) Date of publication and mention of the grant of the patent: **24.11.2004**
(21) Application number: 01934757.4
(22) Date of filing: 18.05.2001
(51) Int. Cl.: C11D 1/66, C11D 17/00, B01F 17/00, A61K 7/48

(54) **A MICROEMULSION CONTAINING A BRANCHED ALKYL GLYCOSIDE**
VERZWEIGTES ALKYLGLYKOSID ENTHALTENDE MIKROEMULSION
MICROEMULSION CONTENANT UN GLYCOSIDE D'ALKYLE RAMIFIE

(30) Priority: 25.05.2000 SE 0001973
(43) Date of publication of application: 19.03.2003
(73) Proprietor: Akzo Nobel N.V., 6800 SB Arnhem (NL)
(72) Inventor: JOHANSSON, Ingegärd, S-416 54 Göteborg (SE); STRANDBERG, Christine, S-431 67 Mölndal (SE)
(74) Representative: Alferink, Petrus J.T.
(86) International application number: PCT/SE2001/001109
(87) International publication number: WO 2001/090286

(56) References cited:
- EP-A2- 0 507 047
- EP-A2- 0 801 130
- WO-A1-94/21655
- WO-A1-94/21769
- WO-A1-95/13863
- WO-A1-95/31957
- WO-A1-97/18033
- WO-A1-98/22207
- WO-A1-99/08517

## Description

The present invention relates to a one-phase microemulsion that contains an emulsifier system containing a branched alkyl glycoside and optionally a linear alkyl glycoside, a water insoluble oil component, water and, if needed, minor amounts of a cosurfactant/cosolvent. This microemulsion may be used e.g. in a cleaning process for removal of hydrophobic substances from hard surfaces or textile, and in personal care or pharmaceutical formulations.

A microemulsion is defined as a thermodynamically stable, macroscopically homogeneous mixture of oil, water and surfactant. It contains, on a microscopical level, individual domains of oil and water separated by a surfactant layer. The visual appearance may be very similar to a true solution but also somewhat opaque or bluish depending on the microscopic structure. This structure has a high degree of dynamics thus making it irrelevant to talk about droplet size and to refer to a microemulsion as an emulsion with micro-droplets. The decisive property is the thermodynamic stability. For a general description of microemulsions and their properties see "Surfactants and polymers in aqueous solution", Jönsson B., Lindman B., Holmberg K., Kronberg B., Wiley&Sons Ltd, 1998, 365-399.

Microemulsions can be used technically in different ways
1. To solubilize hydrophobic components from a surface, i.e. cleaning of oily dirt from hard surfaces, from textile, from skin, from rocks, from soil etc.
2. To carry water insoluble ingredients in a practical form i.e. in personal care formulations, pharmaceutical formulations, for agrochemical additives.

Traditionally, classical nonionics (alkyl alkoxylates) have been used as emulsifiers for microemulsions in combination with cosurfactants or cosolvents, such as more or less short-chained alcohols or glycols.

However, the temperature and electrolyte sensitivity of the alkoxylates, giving rise to phase separation (cloud points), create difficulties in the practical applications.

The choice of alkyl glycosides, being more robust against changes both in temperature and electrolyte content, as emulsifier to overcome these problems, has been shown to be beneficial in a number of patent publications. The general use of glycosides in microemulsions has been described several years ago both in scientific papers (Fukuda et al, Langmuir, 1993, 9, 2921) and in specific applications (Luders H., Balzer D., Proc.2^{nd} World Surfactant Congr. 1988, Paris, Vol II, 81-93).

In the early practical uses, cosolvents like C3-C6 alcohols, alkyl glycols, alkyl oligoglycols or amines were used, which are said to create problems with smell, aggressiveness to skin. In EP 801 130 alkyl polyglycoside is used as an emulsifier with a possible cosurfactant of conventional anionic, cationic or betaine character. Included in the microemulsion are cosolvents in the form of esters of polybasic carboxylic acids with C1-C4 alcohols, thus providing an alternative to the cosolvents mentioned above.

A similar solution is suggested in WO 95/31957, using an emulsifier system consisting of alkyl glycoside with a linear C8-C14 alkyl chain and a fatty acid polyol partial ester with an HLB <11 for making microemulsions containing an oil phase of paraffinic type or a dialkyl ether or the mixture of the two. It may also contain electrolyte and an ionic surfactant.

In WO 97/23192 a microemulsion for cleaning purposes with skin contact is disclosed. The oil phase contains rather polar oils, like fatty acid triglycerides and/or mono and/or diesters of mono or dibasic fatty acids with alcohols or diols. The emulsifiers are alkyl glucamides and/or alkyl oligo glycosides with C8-C22 carbon chain length. The cosurfactant is a partial ester of fatty acid with C10-C18 carbon chain length and a polyol.

WO 97/06.776 concerns a microemulsion for use as antiperspirant/deodorant. It contains similar ingredients as the above-mentioned WO 97/23192 and in addition water soluble antiperspirant additives. The system with nonionic emulsifiers, especially from the group of alkyl polyglucosides, and skin friendly, refattening, oils is claimed to be suitable in this application. A lipophilic cosurfactant and other common additives may be added. The problem of a high viscosity as well as electrolyte sensitivity in the microemulsion is pointed out.

It is well known that one-phase microemulsions often contain liquid crystals or form lamellar structures (see chapter 18 in "Surfactants and polymers in aqueous solution" referred to above). This creates problems with phase separation and/or high viscosity. With the linear alkyl polyglycosides used in the prior art this situation is often encountered, and the problem is e.g. solved by adding comparatively large amounts of cosurfactants/cosolvents.

It has astonishingly been found that introducing a branched alkyl glycoside as an emulsifier to obtain a microemulsion can change this behaviour drastically showing no liquid crystal formation and no high viscosity. Even at high concentrations of the branched alkyl glycoside emulsifier and with no addition of cosurfactant/cosolvent, the viscosity of the microemulsion is low. This effect also remains in mixtures with linear alkyl glycosides, which sometimes may be added to facilitate the formation of a one-phase microemulsion.

The one-phase microemulsion of the invention has an oil/water ratio of 1:99 to 60:40 characterised in that it contains an alkyl glycoside emulsifier system which contains a branched alkyl glycoside with the formula

R₁OGₙ (I)

where Ri is a branched alkyl group containing 8-16 carbon atoms, preferably 8-14 carbon atoms, most preferably 8-12 carbon atoms, G is a saccharide residue and n, the degree of polymerisation, is between 1-5, the weight ratio between the emulsifier system and the oil being less than 4:1, preferably less than 2:1, and most preferably less than 1.5:1.

Depending on the components that are present in the microemulsion, the amount of branched alkyl glycoside could constitute 100% of the total amount of the alkyl glycoside emulsifier system, but for some microemulsions it may be suitable to add a linear alkyl glycoside according to formula

R₂OGₙ (II)

where R₂ is a linear alkyl group containing 6-18 carbon atoms, preferably 6-12 carbon atoms, G is a saccharide residue and n is between 1-5, in an amount of up to 70%, preferably from 5 to 60% by weight of the total amount of the alkyl glycoside emulsifier system. Still, for other microemulsions the amount of linear alkyl glycoside may be between 5 and 45%.

The oil/water ratio range shows that the microemulsion can be in the form of a concentrate or in water diluted form. Furthermore, the one-phase microemulsion requires no, or much less, of a hydrophobic (low HLB) cosurfactant/cosolvent than the corresponding microemulsions containing only linear alkyl glycosides. Since the solubility of such a cosurfactant in the oil phase is changed with temperature, and thus the actual availability of the cosurfactant decreases with increased temperature, the total system is much more temperature stable if little or no cosurfactant is needed to create a microemulsion. It has been found that the amount of cosurfactant needed in the compositions is related to the amount of the linear alkyl glycoside present.

Also by regulating the degree of polymerisation of the alkyl glycosides the amount of cosurfactant or cosolvent can be minimised. Normally the average degree of polymerisation for the alkyl glycoside emulsifier system is between 1. 2 -2. 5, although the degree of polymerisation for certain types of alkyl glycosides included in the system may be outside this range.

Another problem with alkyl glycosides based on longer linear alkyl chains, such as C12, is the high Krafft point, giving rise to unstable microemulsions where the alkyl glycoside separates as crystals at lower temperatures. Using a branched C12-glycoside, the Krafft point is essentially reduced. For example, the Krafft point for n-dodecyl glycoside is 36°C as compared to < 0°C for 2-butyloctyl glycoside. In both cases the degree of polymerization was 1.8.

Since one of the application areas for this type of microemulsions is cleaning of hard surfaces, especially when it comes to very difficult, greasy dirt, the efficiency is dependent on the wetting ability against such a contaminated surface. Also in this aspect it was astonishingly found that the branched alkyl glycosides increase the efficiency, showing much better wetting as such, but also in mixture with linear alkyl glycosides.

Thus, the microemulsion composition of the invention all in all solves the problems described above concerning stability and viscosity. It also exhibits an increased wetting of hydrophobic surfaces as compared to a microemulsion based on only a linear alkyl glycoside as emulsifier.

The oil component in the microemulsion composition according to the invention is water insoluble. Suitable oils may be of the hydrocarbon type, for example cyclohexane, octane, decane, dodecane and different paraffinic cuts like Halpasol 190/240, as well as more polar oils such as long chain alcohols, triglycerides or fatty acid esters and mixtures thereof.

Suitable branched alcohols that could be used to make the branched alkyl glycosides according to the invention may be of the Guerbet type, such as 2-ethylhexanol, 2-propylheptanol, 2-butyloctanol, 2-pentylnonanol, 2-hexyldecanol; 2-ethyloctanol, 2-butylhexanol, 2-butyldecanol and 2-hexyloctanol. Another suitable type is the highly methyl branched (with two or more methyl branches) primary alcohols that are sold under for instance the trade name Exxal, such as Exxal 8, Exxal 9, Exxal 10, Exxal 11, Exxal 12 and Exxal 13. One further type of suitable branched alcohols is represented by the formula 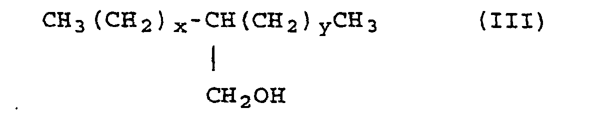 where x and y are at least one and the sum of x+y+4 is the number of carbon atoms in the alcohol. Alcohols of this type are present in the mixtures of linear and monobranched alcohols that are traded as Lials, containing approximately equal amounts of the branched and the linear alcohols. Preferred branched alkyl glycosides are 2-ethylhexyl glucoside, 2-propylheptyl glucoside, 2-butyloctyl glucoside, Lial 111 glucoside, Exxal 10 glucoside, Exxal 11 glucoside and Exxal 12 glucoside. In suitable embodiments of the invention the branched alkyl glycosides are derived from alcohols consisting of at least 50%, preferably at least 65%, of an alcohol of the Guerbet type, a primary alcohol containing at least two methyl branches in the alkyl chain or alcohols of the formula (III) or a mixture thereof.

The linear alkyl glycosides can be made from conventional straight chain alcohols, like hexanol, octanol, decanol and dodecanol.

General production descriptions for alkyl glycosides, and further details concerning the types of possible structures for branched alkyl glycosides can be found for instance in EP-B-690 868 and EP-B-690 905.

In addition, cosurfactants/cosolvents may be present in the microemulsion composition according to the present invention. Typically, these are fatty alcohols or diols with C8-C14 chain length, as well as other hydrophobic straight chain nonionic surfactants with a small hydrophilic head e.g. alkyl glyceryl ethers. The microemulsion composition may also contain short chain glycols like propylene glycol, dipropylenglycol, dipropylenglycol monomethyl ether (DPM) and similar well-known formulation stabilisers.

The microemulsion according to the invention can be obtained with no addition of cosolvent/cosurfactant. When present, the cosurfactant/cosolvent normally amounts to less than 50% by weight of the alkyl glycoside system, preferably less than 35%, and often less than 15%. Nevertheless, higher amounts may be desirable for certain applications.

To the microemulsion could also be added e.g. complexing agents, such as NTA, EDTA, phoshates and citrates, and pH regulating additives, such as alkali hydroxides and silicates, as well as perfumes, pearlescing agents, opacifiers, colors, color stabilisers, preservatives, defoamers and foam boosters.

The choice of oil and additives determines the application area where the invention may be used. With esters, triglycerides and other skin compatible oils it can be used as a mild cleaning agent or as a carrier of active ingredients in the personal care area. The latter functional use can also be found within pharmaceutical applications with edible or non-toxic oils. With good degreasing oils like paraffinic solvents or methyl laurate a hard surface cleaner can be formulated. Other suitable areas are for example agrochemical formulations including active compounds not soluble in water, but in the microemulsion of the invention. Examples of such active compounds are plant growth regulators, herbicides and pesticides, for example insecticides, fungicides or acaricides.

The following embodiments illustrate the invention, and they should not be construed as limiting the scope thereof.

### Example 1

The microemulsions in Table 1 were prepared in the following way. All the components were weighed and mixed in a test tube, left to equilibrate at 50°C approximately half an hour and then shaken thoroughly to ensure that all the components were dissolved properly.

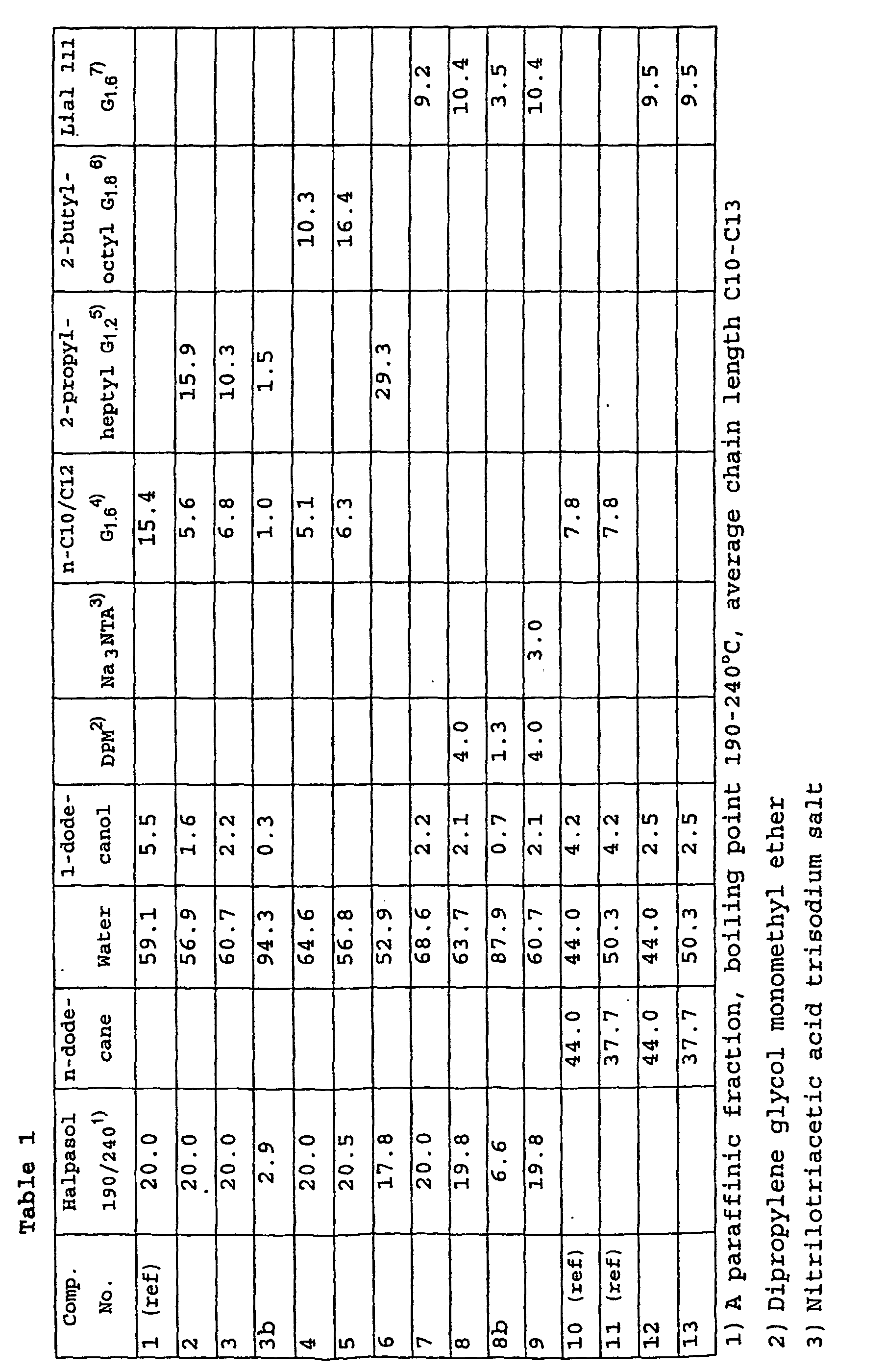

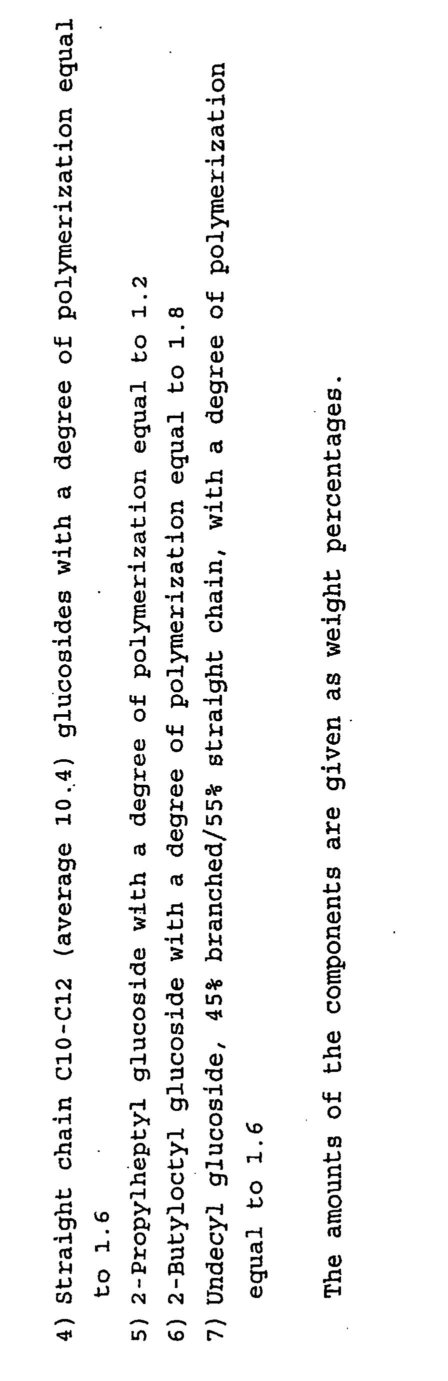

All the microemulsions were clear to opaque. All the compositions according to the invention were one-phase microemulsions, which did not show any optical activity. Sample 1, 10 and 11 showed optical activity, which indicates that lamellar structures are present. Sample 10 and 11 contained liquid crystalline phases and tended to separate with time in two clear microemulsion phases. The pourpoint for sample 1 is 5°C and for sample 2, 3 and 4 it is below -1°C.

From the compositions and appearance of the microemulsions the following conclusions can be drawn.
- The need for relative amounts of cosolvent/cosurfactant (here 1-dodecanol) ranges from 0 to 0.13 (ratio dodecanol/glucoside) when the branched alkyl glucoside is dominating the emulsifier mixture. The more straight chain glucoside that is present, the higher the ratio gets. With only straight chain glucoside the ratio varies between 0.36 (sample 1) and 0.54 (sample 10).
- It is obvious that the appearance of lamellar structures or liquid crystals is only seen with the straight chain glucoside, compare sample 1, 10 and 11 with the others.
- The stability is also good when diluted. Compare samples 3 and 3b as well as 8 and 8b.
- Sample 8 and 9 show that it is easy to include a sequestering agent like nitrilotriacetic acid trisodium salt (Na₃NTA).

### Example 2

The viscosity was measured at room temperature with the following equipment:
Brookfield LVDV-11+ apparatus
Cylindrical sample chamber (19 mm with volumes 2-16 ml) Spindle 18 range 3 - 10000 cP

**Table 2**

| Composition No. | Viscosity (cP) |
|---|---|
| 1 (reference) | 298 |
| 2 | 20.9 |
| 3 | 17.0 |
| 4 | 20.2 |

Composition 1, which contains only straight chain alkyl glucosides as emulsifiers, is approximately 15 times more viscous than the corresponding branched alkyl glucoside compositions.

### Example 3

To investigate the wetting ability of the microemulsions, the following procedure was used:
A lacquered white metal plate is painted with asphalt. The drying time is 24 hours at room temperature. 15 ml of the test sample is poured from one side of the plate, which is placed in an inclined position, and after 1 minute the plate is rinsed with water. The result is evaluated by measuring the wetting capacity, i.e. the width of the cleaned surface, which is measured with a ruler.

**Table 3**

| Composition No. | Width of cleaned surface (cm) |
|---|---|
| 1 (reference) | 5.5 |
| 2 | 6.5 |
| 3 | 6.0 |
| 4 | 7.0 |
| 7 | 5.5 |
| 8 | 7.0 |
| 10 (reference) | 4.6 |
| 11 (reference) | 4.3 |
| 12 | 5.6 |
| 13 | 5.7 |

When comparing the results obtained with the compositions containing the same amount of oil, it is obvious that the microemulsion compositions containing branched chain glucosides give rise to a better wetting than the reference compositions.

## Claims

1. A one-phase microemulsion composition having an oil/water ratio of 1:99 to 60:40 **characterised in that** it contains an alkyl glycoside emulsifier system which contains a branched alkyl glycoside with the formula
R₁OGₙ (I)
where R₁ is a branched alkyl group containing 8-16 carbon atoms, G is a saccharide residue and n, the degree of polymerisation, is between 1-5, the weight ratio between the emulsifier system and the oil being less than 4:1.

2. A microemulsion composition according to claim 1 **characterised in that** it further contains a linear alkyl glycoside with the formula
R₂OGₙ (II)
where R₂ is a linear alkyl group containing 6-18 carbon atoms, G is a saccharide residue and n is between 1-5; where the linear alkyl glycoside is present in an amount of up to 70%(w/w) of the total amount of the alkyl glycoside emulsifier system.

3. A microemulsion composition according to claim 2 **characterised in that** the alkyl glycoside emulsifier system contains 5-60% of the linear alkyl glycoside.

4. A microemulsion composition according to claims 1-3,
where the branched alkyl glycoside is derived from an alcohol consisting of at least 50% of an alcohol of the Guerbet type; a primary alcohol containing at least two methyl branches in the carbon chain; or alcohols of the formula 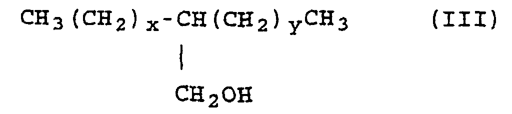 where x and y are at least one and the sum of x+y+4 is the number of carbon atoms in the alcohol; or a mixture thereof.

5. A microemulsion composition according to claims 1-4 where the weight ratio between the emulsifier system and the oil is less than 2:1.

6. A microemulsion according to claims 1-5 where R₁ contains 8-14 carbon atoms.

7. A microemulsion according to claims 1-6 where R₁ contains 8-12 carbon atoms.

8. A microemulsion according to claims 1-7 where R₂ contains 6-12 carbon atoms.

9. A microemulsion according to claims 1-8 where the oil is a water insoluble hydrocarbon or polar oil or a mixture thereof.

10. Use of a microemulsion according to claims 1-9 in a cleaning process for removal of hydrophobic substances.

11. Use of a microemulsion according to claims 1-9 in a personal care formulation.

12. Use of a microemulsion according to claim 10 for cleaning of hard surfaces.

## Patentansprüche

1. Einphasige Mikroemulsionszusammensetzung mit einem Verhältnis Öl/ Wasser von 1:99 bis 60:40, **dadurch gekennzeichnet, dass** sie ein Alkylglycosid-Emulgatorsystem enthält, das ein verzweigtes Alkylglycosid mit der Formel
R₁OGₙ (I)
worin R₁ eine verzweigte Alkylgruppe mit 8 bis 16 Kohlenstoffatomen ist, G ein Saccharidrest ist und n, der Polymerisationsgrad, zwischen 1 - 5 ist, enthält, wobei das Gewichtsverhältnis zwischen dem Emulgatorsystem und dem Öl kleiner als 4:1 ist.

2. Mikroemulsionszusammensetzung nach Anspruch 1, **dadurch gekennzeichnet, dass** sie ferner ein lineares Alkylglycosid mit der Formel
R₂OGₙ (II)
enthält, worin R₂ eine lineare Alkylgruppe mit 6 bis 18 Kohlenstoffatomen ist, G ein Saccharidrest ist und n zwischen 1 - 5 ist, wobei das lineare Alkylglycosid in einer Menge von bis zu 70% (Gew./Gew.) der Gesamtmenge des Alkylglycosid-Emulgatorsystems vorhanden ist.

3. Mikroemulsionszusammensetzung nach Anspruch 2, **dadurch gekennzeichnet, dass** das Alkylglycosid-Emulgatorsystem 5 bis 60% des linearen Alkylglycosids enthält.

4. Mikroemulsionszusammensetzung nach den Ansprüchen 1 bis 3, worin das verzweigte Alkylglycosid abgeleitet ist aus einem Alkohol bestehend aus mindestens 50% eines Alkohols vom Guerbet-Typ, einem primären Alkohol enthaltend mindestens zwei Methylverzweigungen in der Kohlenstoffkette oder Alkoholen der Formel 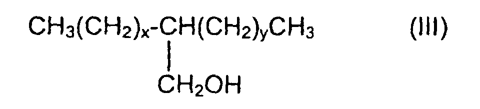 worin x und y mindestens 1 sind und die Summe von x+y+4 die Anzahl der Kohlenstoffatome im Alkohol ist, oder einer Mischung davon.

5. Mikroemulsionszusammensetzung nach den Ansprüchen 1 bis 4, wobei das Gewichtsverhältnis zwischen dem Emulgatorsystem und dem Öl kleiner als 2:1 ist.

6. Mikroemulsion nach den Ansprüchen 1 bis 5, worin R₁ 8 bis 14 Kohlenstoffatome enthält.

7. Mikroemulsion nach den Ansprüchen 1 bis 6, worin R₁ 8 bis 12 Kohlenstoffatome enthält.

8. Mikroemulsion nach den Ansprüchen 1 bis 7, worin R₂ 6 bis 12 Kohlenstoffatome enthält.

9. Mikroemulsion nach den Ansprüchen 1 bis 8, wobei das Öl ein wasserunlöslicher Kohlenwasserstoff oder ein polares Öl oder eine Mischung davon ist.

10. Verwendung einer Mikroemulsion nach den Ansprüchen 1 bis 9 in einem Reinigungsverfahren zur Entfernung von hydrophoben Substanzen.

11. Verwendung einer Mikroemulsion nach den Ansprüchen 1 bis 9 in einer Körperpflegeformulierung.

12. Verwendung einer Mikroemulsion nach Anspruch 10 zur Reinigung von harten Oberflächen.

## Revendications

1. Composition en microémulsion monophasique ayant un rapport huile/eau de 1:99 à 60:40, **caractérisée en ce qu'**elle contient un système émulsifiant à base d'un alkylglycoside, qui contient un alkylglycoside ramifié de formule
R₁OGₙ (I)
dans laquelle R₁ est un groupe alkyle contenant 8 à 16 atomes de carbone, G est un résidu de saccharide et n, le degré de polymérisation, est compris entre 1 et 5, le rapport en poids entre le système émulsifiant et l'huile étant inférieur à 4:1.

2. Composition en microémulsion selon la revendication 1, **caractérisée en ce qu'**elle contient en plus un alkylglycoside linéaire de formule
R₂OGₙ (II)
dans laquelle R₂ est un groupe alkyle linéaire contenant 6 à 18 atomes de carbone, G est un résidu de saccharide et n est compris entre 1 et 5 ; où l'alkylglycoside linéaire est présent en une quantité allant jusqu'à 70 % (m/m) par rapport à la quantité totale du système émulsifiant à base d'un alkylglycoside.

3. Composition en microémulsion selon la revendication 2, **caractérisée en ce que** le système émulsifiant à base d'un alkylglycoside contient 5 à 60 % de l'alkylglycoside linéaire.

4. Composition en microémulsion selon les revendications 1 à 3, dans laquelle l'alkylglycoside ramifié dérive d'un alcool consistant en au moins 50 % d'un alcool de type Guerber ; d'un alcool primaire contenant au moins deux ramifications méthyle dans la chaîne carbonée : ou d'alcools de formule 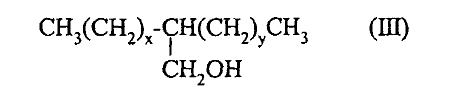 dans laquelle x et y valent chacun au moins 1, et la somme x + y + 4 est le nombre d'atomes de carbone dans l'alcool ; ou d'un mélange de ceux-ci.

5. Composition en microémulsion selon les revendications 1 à 4, dans laquelle le rapport en poids entre le système émulsifiant et l'huile est inférieur à 2:1.

6. Composition en microémulsion selon les revendications 1 à 5, dans laquelle R₁ contient 8 à 14 atomes de carbone.

7. Composition en microémulsion selon les revendications 1 à 6, dans laquelle R₁ contient 8 à 12 atomes de carbone.

8. Composition en microémulsion selon les revendications 1 à 7, dans laquelle R₂ contient 6 à 12 atomes de carbone.

9. Microémulsion selon les revendications 1 à 8, dans laquelle l'huile est une huile hydrocarbonée ou polaire insoluble dans l'eau, ou un mélange de celles-ci.

10. Utilisation d'une microémulsion selon les revendications 1 à 9 dans un procédé de nettoyage, pour éliminer des substances hydrophobes.

11. Utilisation d'une microémulsion selon les revendications 1 à 9 dans une formulation pour les soins corporels.

12. Utilisation d'une microémulsion selon la revendication 10 pour nettoyer des surfaces dures.
